# DEMANDE DE BREVET EUROPEEN

(11) **EP 1 027 884 A1**
(43) Date de publication de la demande: **16.08.2000**
(21) Numéro de dépôt: 00400030.3
(22) Date de dépôt: 07.01.2000
(51) Int. Cl.: A61K 7/50, A61K 7/02

(54) **Composition à usage topique contenant un diol acétylénique et son utilisation pour le nettoyage ou le démaquillage de la peau, des muqueuses et des cheveux**

(30) Priorité: 12.02.1999 FR 9901735
(71) Demandeur: L'OREAL, 75008 Paris (FR)
(72) Inventeur: Simon, Pascal, 94400 Vitry S/Seine (FR)
(74) Mandataire: Rasson, Catherine

(57) **Abrégé**

L'invention concerne une composition à usage topique contenant, dans un milieu physiologiquement acceptable, de 0,01 à 0,5 % en poids par rapport au poids total de la composition, d'un ou plusieurs diols acétyléniques oxyéthylénés de formule (I) : 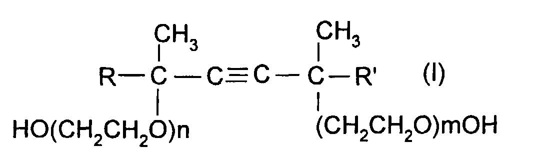 dans laquelle:
- R et R' représentent indépendamment l'un de l'autre un radical alkyle ayant de 1 à 10 atomes de carbone;
- n et m représentent un nombre allant de 0 à 10, et la somme n+m va de 2 à 10, et au moins un composé amphiphile solubilisant du diol acétylénique oxyéthyléné, de HLB égal ou supérieur à 15.

L'invention se rapporte aussi à l'utilisation cosmétique de la dite composition notamment pour le nettoyage et/ou le démaquillage de la peau, des muqueuses et des cheveux.

## Description

L'invention a pour objet une composition à usage topique contenant un diol acétylénique oxyéthyléné, et à son utilisation dans le domaine cosmétique, notamment pour le démaquillage et/ou le nettoyage de la peau, des cheveux et/ou des muqueuses.

L'utilisateur attend d'une composition démaquillante qu'elle permette d'ôter toutes sortes de produits de maquillage: rouge à lèvres, poudre, ombre à paupières, fond de teint etc., sans abîmer la peau et en la laissant propre et douce.

Parmi les produits de démaquillage, beaucoup ont des bases aqueuses: lotions aqueuses, hydroalcooliques, hydroglycoliques, gels aqueux transparents. Ils contiennent des tensioactifs solubilisés qui assurent le démaquillage par la mise en suspension des produits de maquillage. Ces tensioactifs doivent avoir une bonne efficacité de démaquillage et une bonne tolérance pour la peau, et ils doivent assurer une bonne stabilité à la composition les contenant.

Le plus souvent, un tensioactif efficace pour le nettoyage ou le démaquillage, comme par exemple un tensioactif de la classe des anioniques, est mal toléré par la peau. En revanche, un tensioactif doux, comme par exemple un tensioactif non ionique, a souvent une activité détergente insuffisante. Dans le cas de ces tensioactifs non ioniques, on peut rechercher une meilleure efficacité de démaquillage en augmentant leur quantité dans la composition, mais on risque alors de se heurter à des problèmes de stabilité: recristallisation, coloration, mauvaise tenue à la lumière et à la température.

Il subsiste donc le besoin de disposer d'un tensioactif peu irritant et donc non ionique, qui possède toutefois de bonnes propriétés de démaquillage à faible concentration.

Le document US-A-3,268,593 décrit l'utilisation de diols acétyléniques oxyéthylénés comme tensioactifs et détergents. Toutefois, ces diols, pour être efficaces, doivent être utilisés en des quantités qui peuvent entraîner des intolérances pour la peau.

La demanderesse a découvert que parmi ces diols acétyléniques oxyéthylénés, ceux comportant un petit nombre de groupes oxyéthylénés étaient les plus efficaces, même en une faible concentration, pour le démaquillage et/ou le nettoyage de la peau, des muqueuses et/ou des cheveux. Toutefois, tels quels, ces diols faiblement oxyéthylénés peuvent être difficilement incorporés dans des compositions topiques du fait de leur faible solubilité dans l'eau. La demanderesse a trouvé qu'en les associant à un composé amphiphile de HLB égal ou supérieur à 15, on pouvait facilement les incorporer dans une composition topique.

L'invention a donc pour objet une composition à usage topique, contenant, dans un milieu physiologiquement acceptable, de 0,01 à 0,5 % en poids par rapport au poids total de la composition, d'un ou plusieurs diols acétyléniques oxyéthylénés de formule (I): 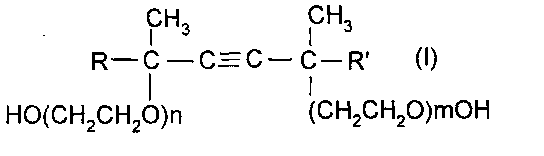 dans laquelle:
- R et R' représentent indépendamment l'un de l'autre un radical alkyle ayant de 1 à 10 atomes de carbone;
- n et m représentent un nombre allant de 0 à 10, et la somme n+m va de 2 à 10, et au moins un composé amphiphile solubilisant du diol acétylénique oxyéthyléné, de HLB égal ou supérieur à 15.

Les composés de formule (I) permettent d'obtenir des compositions qui sont durablement stables et très bien tolérées notamment du fait de l'efficacité de ces composés à de faibles pourcentages. En outre, ces composés possèdent en eux-mêmes une bonne efficacité de démaquillage et de nettoyage à de faibles pourcentages.

Dans la formule (I), les radicaux R ou R' peuvent représenter par exemple un radical éthyle, méthyle, propyle, isopropyle, butyle et/ou isobutyle. Selon un mode préféré de réalisation de l'invention, R et R' sont identiques.

Dans le composé de formule (I), n et m peuvent être des nombres entiers ou non entiers.

Selon une forme préférée de réalisation de l'invention, le diol de formule (I) est choisi parmi les dérivés oxyéthylénés du tétraméthyl-2,4,7,9 décyne-5 diol-4,7 (formule (I) avec R=R'= isopropyl), commercialisés par la société Air Products sous le nom de Surfynol, et plus spécialement le tétraméthyl-2,4,7,9 décyne-5 diol-4,7 oxyéthyléné à 3,5 moles d'oxyde d'éthylène commercialisé sous le nom de Surfynol 440.

La quantité du diol acétylénique oxyéthyléné dans la composition de l'invention va de 0,01 à 0,5 % et de préférence de 0,05 à 0,5 % en poids par rapport au poids total de la composition.

Le composé amphiphile solubilisant du diol acétylénique oxyéthyléné peut être choisi notamment parmi les composés qui ont une solubilité dans l'eau allant jusqu'à au moins 5 % en poids. Un tel composé a un HLB (Balance Hydrophile Lipophile) égal ou supérieur à 15 et peut être ionique, non ionique ou amphotère. Il s'agit de préférence d'un composé non ionique.

Comme composé amphiphile non ionique utilisable dans la composition de l'invention, on peut citer notamment:
1) les alkényl succinates alkoxylés ou glycérolés, tels que l'hexadécényl succinate 18 OE commercialisé par la société ICI sous la dénomination Arlatone 8016 et le dodécénylsuccinate de polyglycérol 3 OE commercialisé par la société BASF sous la dénomination Cremophor COS;
2) les copolymères blocs d'oxyde d'éthyléne et d'oxyde de propylène, tels que les Poloxamer;
3) les éthers d'alcool gras et de sucre, tels que les alkylpolyglucosides;
4) les esters gras de sorbitan et les esters gras de sorbitan oxyéthylénés tels que le laurate de sorbitan 20 OE (polysorbate 20);
5) les esters de glycérol oxyéthylénés tels que le triester d'acide acétique et de glycereth-7 (glycereth-7 triacetate),
et leurs mélanges.

La quantité de composé amphiphile solubilisant peut varier dans une large mesure suivant la quantité de diol acétylénique présent dans la composition et elle peut aller par exemple de 0,001 à 10 % et de préférence de 0,01 à 5 % en poids par rapport au poids total de la composition. Selon un mode préféré de réalisation de l'invention, le rapport en poids entre le diol acétylénique oxyéthyléné et le composé amphiphile solubilisant va de préférence de 1:10 à 10:1 et mieux de 1:2 à 2:1.

La composition de l'invention destinée à une application topique contient un milieu physiologiquement acceptable, c'est-à-dire compatible avec la peau y compris le cuir chevelu, les muqueuses et/ou les yeux et peut constituer notamment une composition cosmétique et/ou dermatologique.

Cette composition peut se présenter sous toutes les formes galéniques normalement utilisées dans les domaines cosmétique et dermatologique, et elle peut être notamment sous forme d'une solution aqueuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles.

La composition de l'invention peut constituer par exemple une lotion, un gel ou un lait, et par exemple une lotion ou un lait de démaquillage ou de nettoyage, un shampooing ou un gel douche.

La composition selon l'invention peut comprendre une phase grasse à base d'huile animale, végétale, minérale, siliconée, fluorée et/ou synthétique. La phase grasse peut contenir en outre tout autre corps gras susceptible d'être présent dans la phase grasse comme par exemple les acides gras, les alcools gras et les cires comme la cire d'abeille.

On peut notamment citer comme composants de la phase grasse, l'huile de paraffine, l'huile de vaseline, le perhydrosqualène, les huiles végétales (d'amande douce, de palme, de ricin, d'avocat, de jojoba), les huiles de silicone telles que les polydiméthylsiloxanes, éventuellement phénylées telles que les phényltriméthicones, et les huiles de silicone volatiles telles que les cyclomethicones.

La phase grasse peut également comprendre une huile démaquillante telle qu'un ester d'acide gras, en particulier les esters obtenus à partir d'un alcool à chaîne droite ou ramifiée ayant de 1 à 17 atomes de carbone et d'un acide gras à chaîne droite ou ramifiée ayant de 3 à 18 atomes de carbone. Un tel ester peut en particulier être choisi dans le groupe constitué par l'adipate de dioctyle, le palmitate d'octyle (ou d'éthyl-2 hexyle), l'adipate de diisopropyle, l'hexanoate d'octyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'octyle, l'octanoate d'octyle, le caprate/caprylate d'octyle, le palmitate de méthyle, le myristate de butyle, le myristate d'isobutyle, le palmitate d'éthyle, le laurate d'isohexyle, le laurate d'hexyle, l'isostéarate d'isopropyle, l'isononanoate d'isononyle.

La phase grasse peut être présente en une quantité allant par exemple de 1 à 95 % et de préférence de 5 à 30 % en poids par rapport au poids total de la composition dans le cas d'une émulsion.

La composition selon l'invention peut comprendre, en outre,
- un agent permettant la mise en suspension de la phase grasse, par exemple un copolymère d'acrylates d'alkyle en C₁₀-C₃₀ et d'acide acrylique ou méthacrylique ou de leur ester (Pemulen TR1, Pemulen TR2, Carbopol 1342 de la société GOODRICH); ou un copolymère acrylamide/acide méthylpropane sulfonique (Sepigel de la société SEPPIC), et/ou
- un agent de mise en dispersion de la phase grasse, tel qu'un système émulsionnant ou vésiculaire à base de vésicules, éventuellement de taille nanométrique, constituées de lipides ioniques (liposomes) ou non ioniques.

La composition de l'invention peut comprendre, en outre, un agent pour modifier sa viscosité, et obtenir des textures plus ou moins gélifiées, tel que:
- les dérivés cellulosiques (carboxyméthylcellulose, hydroxyéthylcellulose, hydroxypropylméthylcelluose);
- les gommes naturelles telles que les gommes de xanthane, de guar, de caroube, les carraghénanes;
- les dérivés polycarboxyvinyliques du type Carbomer (vendues par la société GOODRICH sous les dénominations Carbopol 940, 951, 980, ou par la société 3V-SIGMA sous la dénomination Synthalen K ou Synthalen L).

La composition selon l'invention peut également comprendre, de façon connue, des adjuvants couramment utilisés dans les domaines considérés, tels que les conservateurs, les antioxydants, les parfums, les charges, les pigments, les filtres UV, les séquestrants (EDTA), les huiles essentielles, les matières colorantes, les solvants (comme les alcools inférieurs ayant de 1 à 4 atomes de carbone, tels que l'éthanol), les actifs hydrophiles ou lipophiles tels que les hydratants, notamment les polyols (glycérine, butylène glycol, hexylène glycol), les vitamines et leurs dérivés.

Bien entendu, l'homme du métier veillera à choisir ce ou ces éventuels composés complémentaires et/ou leur quantité de manière telle que les propriétés avantageuses de la composition selon l'invention ne soient pas, ou substantiellement pas, altérées par l'adjonction envisagée.

La composition présente de préférence un pH qui respecte la peau, généralement compris entre 5 et 8, de préférence un pH de 5,5 à 7,5.

La composition de l'invention est particulièrement efficace pour le nettoyage et/ou le démaquillage de la peau, des muqueuses (lèvres), des phanères telles que les cheveux et/ou des yeux. et elle a l'avantage de ne pas être non irritante. Elle peut constituer en particulier un lait ou une lotion de nettoyage et/ou de démaquillage pour les yeux, les lèvres et/ou la peau, notamment la peau du visage, un shampooing et/ou un gel douche.

Un autre objet de l'invention est l'utilisation cosmétique de la composition telle que définie ci-dessus pour le nettoyage et/ou le démaquillage de la peau, des muqueuses (lèvres), des cheveux et/ou des yeux.

Un autre objet de l'invention est un procédé cosmétique de nettoyage et/ou de démaquillage de la peau, des muqueuses, des cheveux et/ou des yeux, caractérisé en ce qu'on applique sur la peau, les muqueuses, les cheveux et/ou les yeux, une composition telle que définie ci-dessus.

Les exemples qui suivent permettront de mieux comprendre l'invention, sans toutefois présenter un caractère limitatif. Les quantités indiquées sont en % en poids.

### Exemple 1 : Lotion

| | |
|---|---|
| - Tétraméthyl-2,4,7,9 décyne-5 diol-4,7 oxyéthyléné à 3,5 moles d'oxyde d'éthylène (Surfynol 440) | 0,1 % |
| - Hexadécényl succinate 18 OE (Arlatone 8016) | 0,1 % |
| - Hexylène glycol | 1 % |
| - EDTA | 0,1 % |
| - Conservateurs | 0,3 % |
| -Eau | qsp 100 % |

Mode opératoire : On dissout les constituants un à un dans l'eau à une température d'environ 75 à 80°C. On laisse refroidir sous faible agitation, puis on complète par de l'eau à qsp 100 %.

On obtient un lotion particulièrement agréable à utiliser et présentant de très bonnes propriétés démaquillantes sans agresser ni irriter la peau qui reste douce.

### Exemple comparatif 1 : Lotion

| | |
|---|---|
| - Sodium laureth sulfate / magnesium laureth sulfate / sodium laureth-8 sulfate / magnesium laureth-8 sulfate (Empicol BSD 52 FL2 à 26 % de matière active commercialisé par la société Albright & Wilson) | 1 % |
| - Disodium cocamphodiacétate (Miranol C2M à 38 % de matière active) commercialisé par la société Rhodia Chimie) | 1,1 % |
| - Hexylène glycol | 1 % |
| - EDTA | 0,1 % |
| - Conservateurs | 0,3 % |
| -Eau | qsp 100 % |

Les lotions de l'exemple 1 et de l'exemple comparatif 1 ont été testées pour le démaquillage des yeux sur un panel de 10 personnes. Bien que contenant beaucoup moins de tensioactif la lotion de l'exemple 1 selon l'invention s'est avérée avoir un pouvoir démaquillant identique à celui de la lotion de l'exemple comparatif. En outre, la lotion de l'exemple 1 est beaucoup plus agréable à utiliser que celle de l'exemple comparatif 1 car elle ne mousse pas, ne laisse pas de film collant sur les paupières et les cils et n'est pas du tout irritante pour les yeux.

### Exemple 2 : Gel démaquillant

| | |
|---|---|
| - Tétraméthyl-2,4,7,9 décyne-5 diol-4,7 oxyéthyléné à 3,5 moles d'oxyde d'éthylène (Surfynol 440) | 0,2 % |
| - Poloxamer 188 (Lutrol F68 commercialisé par la société BASF) | 0,1 % |
| - Acrylates/C10-30 Alkyl crosspolymer (Pemulen TR2 commercialisé par la société Goodrich) | 0,25 % |
| -Soude | 0,01% |
| - Conservateur | 0,1 % |
| -Eau | qsp 100 % |

Mode opératoire : On dissout les constituants un à un dans l'eau à une température d'environ 75 à 80°C, en ajoutant en dernier le Pemulen TR2 et la soude. On laisse refroidir sous faible agitation, puis on complète par de l'eau à qsp 100 %.

### Exemple 3 : Lait démaquillant

| | |
|---|---|
| - Tétraméthyl-2,4,7,9 décyne-5 diol-4,7 oxyéthyléné à 3,5 moles d'oxyde d'éthylène (Surfynol 440) | 0,2 % |
| - Polysorbate 20 | 0,1 % |
| - Acrylates/C 10-30 Alkyl crosspolymer (Pemulen TR2 commercialisé par la société Goodrich) | 0,25 % |
| -Soude | 0,05 % |
| - Palmitate d'octyle | 10 % |
| - Conservateur | 0,1 % |
| -Eau | qsp 100 % |

Mode opératoire On dissout les constituants aqueux un à un dans l'eau à une température d'environ 75 à 80°C, en ajoutant en dernier le Pemulen TR2 et la soude. Puis on ajoute l'huile que l'on émulsionne dans le mélange aqueux. On laisse refroidir sous faible agitation, puis on complète par de l'eau à qsp 100 %.

On obtient un lait permettant un bon démaquillage des cils recouverts de mascaras waterproof ou non waterproof.

## Revendications

1. Composition à usage topique, contenant, dans un milieu physiologiquement acceptable, de 0,01 à 0,5 % en poids par rapport au poids total de la composition, d'un ou plusieurs diols acétyléniques oxyéthylénés de formule (I): 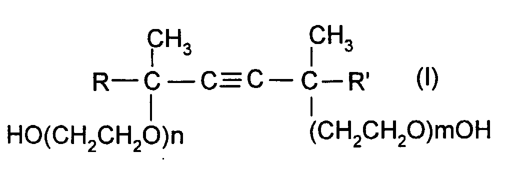 dans laquelle:
- R et R' représentent indépendamment l'un de l'autre un radical alkyle ayant de 1 à 10 atomes de carbone;
- n et m représentent un nombre allant de 0 à 10, et la somme n+m allant de 2 à 10,
et au moins un composé amphiphile solubilisant du diol acétylénique, de HLB égal ou supérieur à 15.

2. Composition selon la revendication 1, caractérisée en ce que les radicaux R et R' dans la formule (I) représentent un radical éthyle, méthyle, propyle, isopropyle, butyle et/ou isobutyle.

3. Composition selon la revendication 1 ou 2, caractérisée en ce que le diol de formule (I) est choisi parmi les dérivés oxyéthylénés du tétraméthyl-2,4,7,9 décyne-5 diol-4,7.

4. Composition selon l'une quelconque des revendications précédentes, caractérisée en ce que le diol acétylénique oxyéthyléné est présent en une quantité allant de 0,05 à 0,5 % en poids par rapport au poids total de la composition.

5. Composition selon l'une quelconque des revendications précédentes, caractérisée en ce que le composé amphiphile est non ionique.

6. Composition selon l'une quelconque des revendications précédentes, caractérisée en ce que le composé amphiphile est choisi parmi les alkényl succinates alkoxylés ou glycérolés, les copolyméres blocs d'oxyde d'éthylène et d'oxyde de propylène, les éthers d'alcool gras et de sucre, les esters gras de sorbitan et les esters gras de sorbitan oxyéthylénés, les esters de glycérol oxyéthylénés et leurs mélanges.

7. Composition selon l'une quelconque des revendications précédentes, caractérisée en ce que le composé amphiphile est présent en une quantité allant de 0,001 à 10 % en poids par rapport au poids total de la composition.

8. Composition selon l'une quelconque des revendications précédentes caractérisée en ce qu'elle contient en outre une phase grasse.

9. Composition selon l'une quelconque des revendications précédentes, caractérisée en ce qu'elle est sous la forme d'une solution, d'une dispersion, d'une émulsion ou d'une dispersion vésiculaire.

10. Composition selon l'une quelconque des revendications précédentes, caractérisée en ce qu'elle constitue une lotion, un gel, un lait, un shampooing.

11. Utilisation cosmétique de la composition selon l'une quelconque des revendications 1 à 10, pour le nettoyage et/ou le démaquillage de la peau, des muqueuses, des cheveux et/ou des yeux.

12. Procédé cosmétique de nettoyage et/ou de démaquillage de la peau, des muqueuses, des cheveux et/ou des yeux, caractérisé en ce qu'on applique sur la peau, les muqueuses, les cheveux et/ou les yeux, une composition selon l'une quelconque des revendications 1 à 10.
